# EUROPEAN PATENT APPLICATION

(11) **EP 2 490 029 A1**
(43) Date of publication of application: **22.08.2012**
(21) Application number: 11154652.9
(22) Date of filing: 16.02.2011
(51) Int. Cl.: G01N 33/94

(54) **Vancomycin analysis**

(71) Applicant: Université Catholique de Louvain, 1348 Louvain-La-Neuve (BE); Université de Liège, 4031 Angleur (BE); Université de Mons, 7000 Mons (BE); Université Libre de Bruxelles, 1050 Bruxelles (BE)
(72) Inventor: Marchand, Jacqueline, 6001, Marcinelle (BE); Tulkens, Paul, 1404, Nivelles (BE); Goormaghtigh, Erik, 7850, Enghien (BE); De Coninck, Joël, 7022, Mesvin (BE); Joris, Bernard, 4141, Grivegnée (BE); Carryn, Stéphane, 1930, Zaventem (BE)
(74) Representative: Bounaga, Sakina

(57) **Abstract**

The present invention relates to a method for detecting and measuring free vancomycin in a sample, comprising the steps of:
(a) providing a device comprising at least one surface which is chemically activated and grafted with a compound able to immobilize vancomycin,
(b) providing a sample, treating said sample with an aqueous solution and adding said treated sample to said device, and
(c) measuring the free vancomycin concentration in said treated sample,
wherein said aqueous solution comprises at least one carbohydrate and is free of the phosphate salts selected from the group M¹H₂PO₄, M¹₂HPO₄, M²₃PO₄, M²(H₂PO₄)₂, M²₃(PO₄)₂ and M²HPO₄, wherein M¹ is Na, Li, or K, and M² is Ca or Mg.

## Description

### Field of the invention

The invention relates to an improved method for detecting and quantifying vancomycin. The invention is particularly important to the monitoring and adjusting of a vancomycin dosage provided to a subject suffering from an infection treatable with vancomycin. This is of interest to the field of clinical biology and medicine.

### Background of the invention

Vancomycin, the most widely used member of the glycopeptides family, has become the standard of therapy to treat infections caused by Gram (+) organisms resistant to beta-lactam antibiotics. Vancomycin is especially useful in the treatment of methicillin resistant Staphylococcus aureus (MRSA) and other staphylococci. The dramatic increase of nosocomial infections in hospitals due to these organisms has resulted in a commensurate increase in vancomycin use.

The therapeutic window of vancomycin is rather narrow. This requires treatments with vancomycin be closely monitored (i) to ensure a sufficiently high serum level is achieved in a subject to cope with the susceptibility of the offending organism; and (ii) to avoid undue toxicity. This implies (i) the adjustment of the doses delivered to the patients, and (ii) the monitoring of the serum ratios to make sure of reaching, but not passing beyond, the wished amounts of drugs. Clinical failures have been reported that were due to sub-therapeutic posologies. On the other hand, too high levels of vancomycin in serum of patients critically increase the risks of toxicity.

As a means to optimize vancomycin therapies, methods of continuous infusion have been selected. This facilitates the monitoring process. However, it requires that vancomycin levels in serum can be readily monitored so that the therapeutic dose can be followed and, if required, adjusted.

Commercial methods for the quantitative analysis of vancomycin are based mainly on an immunological recognition, coupled with the measurement of the amount of drug bound to the corresponding antibodies. Such methods measure the total vancomycin, protein-bound and protein-unbound, present in a sample. However, it is the protein-unbound or free antibiotic, i.e. vancomycin not bound to serum proteins, which is the active drug. In addition, it is reported that the amount of free vancomycin can not be reliably predicted from this assay of the total vancomycin serum concentration.

Laboratory methods have been reported, where vancomycin is immobilized on a support adapted for signal transduction and submitted to recognition by a peptide probe in solution. In order for such a biosensor method to be of value in clinical practice, it should be able to recognize vancomycin present in a biological sample.

In the production and purification of vancomycin, chromatographic methods such as affinity chromatography are used. In this binding-type of method, the peptide probes are immobilized and vancomycin is in solution. Typically a saline solution or buffer such as phosphate buffered saline (PBS) are used a sample media. The replacement of chromatography columns takes time and is expensive.

Therefore, there exists a need for improved methods for vancomycin analysis. It is the aim of the invention to provide a method for the detection and measurement of vancomycin that is applicable to clinical practice. In particular, it is an object of the invention to provide a method that allows repeated blood level monitoring and allows rapid delivery of the results to the clinician to ensure optimal efficacy and avoid undue toxicity.

### Summary of the invention

The method of the invention provides biosensor technology adapted to the detection and measurement, including quantitative analysis, of vancomycin.

In a first aspect, the present invention provides a method for detecting and measuring free vancomycin in a sample, comprising the steps of:
(a) providing a device comprising at least one surface which is chemically activated and grafted with a molecule able to immobilize vancomycin,
(b) providing a sample, treating said sample with an aqueous solution and adding said treated sample to said device, and
(c) measuring the free vancomycin concentration in said treated sample,
wherein said aqueous solution comprises at least one carbohydrate and is free of any of the compounds selected from the group M¹H₂PO₄, M¹₂HPO₄, M¹₃PO₄, M²(H₂PO₄)₂, M²₃(PO₄)₂ and M²HPO₄, wherein M¹ is Na, Li, or K, and M² is Ca or Mg.

The present invention is advantageous as it provides an improved method for the detection and quantification of a commonly used antibiotic. Measuring, monitoring and adjusting of a drug regime are simplified. A method according to an embodiment of the invention allows efficient follow-up of the dosage regime in a patient receiving treatment with vancomycin. This ensures that the dosage delivered to the patient remains within the therapeutic window. Drug effectiveness is improved; side effects due to drug overdosing and toxicity are reduced. A subject's fight to overcome infections may be supported.

The foregoing advantages, as well as other advantages, will be apparent from the detailed description of the invention as set forth herein, the drawings and the examples illustrating it.

### Description of the figures

Figure 1 displays a curve with integrated absorbencies between 1253 and 1218 cm⁻¹ for vancomycin solutions (50 µg/mL) in respectively phosphate buffered saline (PBS), 5% glucose and water. Mean of 10 spectra.

Figure 2 displays a graph with integrated absorbencies between 1253 and 1218 cm⁻¹ for vancomycin solutions (50 µg/mL) in various aqueous media. Mean of 10 spectra.

Figure 3 displays a graph depicting the vancomycin binding curve in 5% glucose. Vancomycin was monitored as the area of the absorption band between 1259 and 1213 cm⁻¹. The data were fitted with a single site binding isotherm, R² of the fit = 0.99. Kd = 13.7 ± 1.8 µM (standard error).

Figure 4 displays a graph depicting the vancomycin binding curve in human serum dialyzed against 5% glucose. Vancomycin was monitored as the area of the absorb absorption band between 1259 and 1213 cm⁻¹. The data were fitted with a single site binding isotherm, R² of the fit = 0.99. Kd = 7.3 +/- 0.8 µM (standard error).

Figure 5 displays a graph depicting the recovery of free vancomycin from serum under conditions of agitation mimicking those that would take place in a blood vessel. All values are given ± Standard Deviation (3 independent tests).

Figure 6 represents a QCM data plot showing delta frequency (MHz) change over time of vancomycin (1 µM and 10 µM) detection in water.

Figure 7 represents QCM data plot showing delta frequency (MHz) change over time of vancomycin (1 µM) detection in water and in glucose.

Figure 8 represents QCM data plot showing delta frequency (MHz) change over time of vancomycin (40µM) detection in water and in glucose.

### Detailed description of the invention

Before the present method and devices used in the invention are described, it is to be understood that this invention is not limited to particular methods, components, or devices described, as such methods, components, and devices may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" also include the term "consisting of".

The recitation of numerical ranges by endpoints includes all numbers and fractions subsumed within the respective ranges, as well as the recited endpoints.

The term "about" as used herein when referring to a measurable value such as a parameter, an amount, a temporal duration, and the like, is meant to encompass variations of +/-10% or less, preferably +/-5% or less, more preferably +/-1 % or less, and still more preferably +/-0.1 % or less of and from the specified value, insofar such variations are appropriate to perform in the disclosed invention. It is to be understood that the value to which the modifier "about" refers is itself also specifically, and preferably, disclosed.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

The present invention relates to an improved method for measuring and/or monitoring the level of free vancomycin in a sample. The present invention provides a method using a device, also referred herein as "biosensor", which is sensitive to vancomycin.

By the term "free vancomycin" it is meant protein unbound vancomycin.

By the term "biosensor" it is meant a device based on the specific recognition of an analyte of interest by a target such as a biological component, for example a receptor, an antibody, an enzyme, a membrane, a cell or cell containing media, a molecule and the subsequent transformation of this interaction into an electrical, optical, or other signal. In the present invention, the term biosensor particularly refers to a device provided to specifically recognize vancomycin with a molecule able to immobilize vancomycin.

By the term "sample" or "biological sample" it is meant a liquid derived from a biological origin, such as from a mammal, for instance from a human or animal. The samples of interest for the detection and quantitative analysis of vancomycin are preferably serum, blood or interstitial fluid or other human fluids susceptible of containing vancomycin. In a preferred embodiment, the invention provides a method as described above wherein the sample is serum, blood or interstitial fluid.

By the term "immobilization" it is meant that the vancomycin and the compound able to immobilize vancomycin strongly interact via several hydrogen-bonds, preferably via at least five hydrogen binding interactions.

According to an embodiment of the invention, the method comprises the steps of: (a) providing a device comprising at least one surface which is chemically activated and covalently grafted with a compound able to immobilize vancomycin, (b) providing a sample, treating said sample with an aqueous solution and adding said treated sample to said device, and (c) measuring the free vancomycin concentration in said treated sample, wherein said aqueous solution comprises at least one carbohydrate and wherein said aqueous solution is free of any of the compounds selected from the group M¹H₂PO₄, M¹₂HPO₄, M¹₃PO₄, M²(H₂PO₄)₂, M²₃(PO₄)₂ and M²HPO₄, wherein M¹ is Na, Li, or K, and M² is Ca or Mg.

In an embodiment, the device used in the method comprises an attenuated total internal reflection element, transparent in the infra-red. In this embodiment, vancomycin is measured with surface sensitive optical methods. In this embodiment, the free vancomycin concentration is measured by Fourier Transform Infra Red (FTIR)-ATR spectroscopy.

According to an embodiment of the invention, the device comprises an attenuated total internal reflection element (ATR), transparent in the infra-red of which at least one surface is chemically activated and covalently grafted with a molecule able to immobilize vancomycin. The ATR configuration allows the study of analytes such as biological components and molecules or proteins, on surfaces in contact with a sample.

In an embodiment, the invention concerns a method detecting and measuring free vancomycin in a sample, comprising the steps of:
(a) providing a device comprising an attenuated total internal reflection element, transparent in the infra-red of which at least one surface is chemically activated and covalently grafted with a compound able to immobilize vancomycin,
(b) providing a sample, treating said sample with an aqueous solution and adding said treated sample to said device, and
(c) measuring the free vancomycin concentration in said treated sample,
wherein said aqueous solution comprises at least one carbohydrate and is free of any of the compounds selected from the group M¹H₂PO₄, M¹₂HPO₄, M¹₃PO₄, M²(H₂PO₄)₂, M²₃(PO₄)₂ and M²HPO₄, wherein M¹ is Na, Li, or K, and M² is Ca or Mg.

In this embodiment, a purposely modified ATR element is provided to study vancomycin-receptor interactions occurring at the solvent ATR element interface, particularly, at the water-containing media-ATR element interface, by using attenuated total internal reflection (ATR) infra-red (IR) spectroscopy, preferably Fourier transform infra-red spectroscopy (FTIR).

In an embodiment of the present invention, the device used for detecting and measuring vancomycin, is preferably based on FTIR-ATR technology. Fourier transform Infra-Red (FTIR) spectroscopy is an extremely powerful analytical technique, particularly well adapted to the characterization of organic molecules and biological systems. Quantitative structural and conformational information can be recorded. In a further embodiment, the invention therefore provides a method as described above wherein the binding of free vancomycin to said receptor is monitored by FTIR-ATR spectroscopy.

In one embodiment, the ATR element is made of a material selected from the group consisting of silicon, germanium, ZnS, ZnSe, or diamond. Preferably, the ATR element is made of germanium or silicon and more preferably the ATR element is made of germanium.

In another embodiment, the ATR element can have any shape as long as it allows internal reflection of a radiation within said ATR element. Preferably, the ATR element is a crystal having a trapezoidal, hemi-cylindrical, rectangular or triangular polyhedral form, rectangular prism, or a triangular prism (prism with triangular basis). More preferably, the ATR element is a triangular prism with a right triangular basis (also called right-angled triangle or rectangled triangle). More preferably, the ATR element is a triangular prism with 45-45-90 triangle basis (right triangle with the two other angles at 45°).

In other embodiments, the device which can be used in the method can be a surface Plasmon resonance sensor such as the BIACORE/Surface Plasmon Resonance sensor. In yet another embodiment, the device used in the method is a quartz crystal microbalance. In this embodiment, the vancomycin is measured with surface sensitive acoustic waveguide techniques.

As used herein "quartz crystal microbalance" (QCM) refers a mass-sensing device. An electrical signal is sent through a gold-plated quartz crystal, producing a vibration at a resonance frequency. Changes in frequency are related to changes in mass on the surface of the crystal. The change in frequency is directly proportional to the mass on the crystal. In the present invention QCMs function as biosensors. A compound able to immobilize vancomycin is associated with the QCM surface. Subsequent binding of the vancomycin results in a measurable change in the resonance frequency.

In this embodiment, the invention concerns a method detecting and measuring free vancomycin in a sample, comprising the steps of:
(a) providing a quartz crystal microbalance of which at least one surface is chemically activated and grafted with a compound able to immobilize vancomycin,
(b) providing a sample, treating said sample with an aqueous solution and adding said treated sample to said device, and
(c) measuring the free vancomycin concentration in said treated sample,
wherein said aqueous solution comprises at least one carbohydrate and is free of any of the compounds selected from the group M¹H₂PO₄, M¹₂HPO₄, M¹₃PO₄, M²(H₂PO₄)₂, M²₃(PO₄)₂ and M²HPO₄, wherein M¹ is Na, Li, or K, and M² is Ca or Mg.

According to the invention, the device comprises an ATR element or a gold-coated quartz crystal, of which at least one surface is chemically activated and covalently grafted with a compound able to immobilize vancomycin. In an embodiment, said compound able to immobilize vancomycin comprises a receptor able to immobilize protein-unbound vancomycin. In an embodiment said receptor is of the general formula X-D-Alanyl-D-Alanine or stereoisomer thereof, wherein
X is NH₂-[(CH₂)ₘ-O]_{y}-(CHR¹)_{z}-CO-L¹-,
m is an integer selected from 1 to 6, for example m is 1, 2, 3, 4, 5, 6,
y is an integer selected from 0 and 10, for example y is 0, 1, 2, 3, 4, 5, 6, 7, 8, 9, 10, preferably 0, 3 or 6,
z is an integer selected from 1 to 10, for example z is 1, 2, 3, 4, 5, 6, 7, 8, 9, 10, preferably 1,
R¹ is H or NHR²,
R² is H or C₁-C₆ alkylcarbonyl, for example C₁-C₅alkylcarbonyl, C₁-C₄alkylcarbonyl, C₁-C₃alkylcarbonyl, C₃alkylcarbonyl, C₂alkylcarbonyl, C₅alkylcarbonyl, or C₆alkylcarbonyl,
and
L¹ is a single bond or -NH-(CH₂)ₙ-CO-, wherein n is an integer selected from 1 to 8, for example n is 1, 2, 3, 4, 5, 6, 7, 8; preferably 5.

In a particular embodiment m is 2. In a particular embodiment y is 0, 3 or 6. In a more particular embodiment m is 2 and y is 3. In another more particular embodiment m is 2 and y is 6. In a particular embodiment z is 1. In a particular embodiment z is 1, m is 2 and y is 3. In another particular embodiment z is 1, m is 2 and y is 6. In a preferred embodiment L¹ is -NH-(CH₂)ₙ-CO-, wherein n is 5.

In an embodiment, X is selected from H₂N-(CH₂)₅-CO-, H₂N-(CH₂-CH₂-O)₃-CH₂-CO-NH-(CH₂)₅-CO-, H₂N-(CH₂-CH₂-O)₆-CH₂-CO-NH-(CH₂)₅-CO-, or L-Lysyl or a protected form thereof. In a preferred embodiment, the invention provides a method as described above wherein X is L-Lysyl, or a protected form thereof, preferably X is N-α-acetyl-L-lysyl. In a preferred embodiment the receptor is N-α-acetyl-L-Lysyl-D-Ala-D-Ala.

Compounds of formula (1), (2), (3) or (4) are non-limiting examples of suitable structures for use as receptor of formula X-D-Ala-D-Ala.

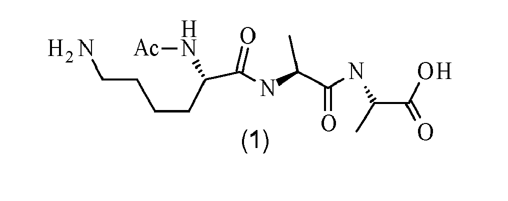

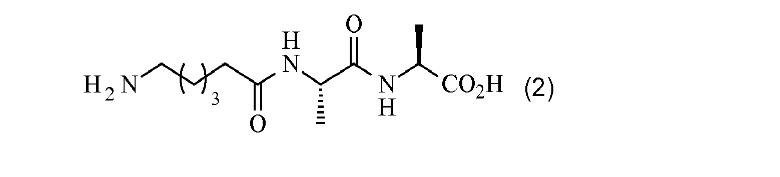

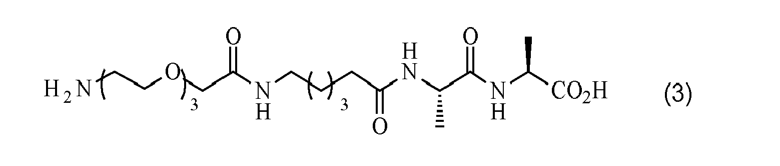

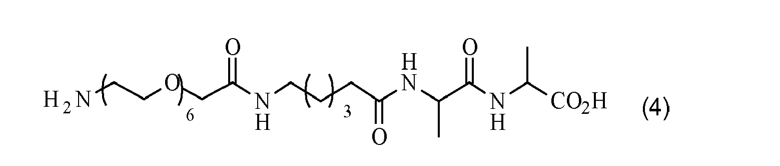

In an embodiment, the receptor of formula X-D-Alanyl-D-Alanine, is grafted, preferably covalently grafted, on the surface of the ATR element or on the gold-plated quartz crystal, via an organic molecule (I) selected from the group comprising HS-(CH₂-CH₂-O)_{w-}CH₃, X¹₃Si-(CH₂)_{q}-NH-CO-(O-(CH₂)ₛ-X¹, CH≡CR⁶, CH₂=CHR⁶, X¹₃Si-(CH₂)_{q}-(CF₂)ₛY¹, X¹₂(R³)Si-(CH₂)_{q}-(CF₂)ₛY¹; or X¹(R³)(R⁴)Si-(CH₂)_{q}-(CF₂)ₛ-Y¹ , wherein
R⁶ is selected from C_{w}H_{2w+1}, C_{w}F_{2w+1}, -(CH₂)ᵤ-(O-CH₂-CH₂)ₚ-OR⁵, wherein R⁵ is selected from C₁-₄alkyl, C₁-₆alkylarylsulfoxide, heteroaryloxycarbonylC₁-₆alkyl, 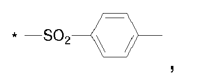 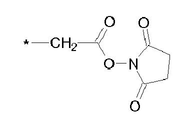 , or mixture thereof,
w is an integer from 3 to 50,
u is an integer from 0 to 20,
p is an integer from 3 to 20;
X¹ is halogen or C₁-₆alkoxy;
q is an integer selected from 1 to 20;
s is an integer selected from 0 to 20;
R³ and R⁴ are each independently C₁-₆alkyl; and
Y¹ is Me, CF₃, CHF₂, CH₂F, CH=CH₂, CN, CH=O, epoxide, halogen, SH, NH₂, OH, N=C=O, N=C=S, CO₂H or derived esters thereof,

As used herein, the term "alkyl" by itself or as part of another substituent, refers to a straight or branched saturated hydrocarbon group joined by single carbon-carbon bonds having 1 to 10 carbon atoms, for example 1 to 8 carbon atoms, for example 1 to 6 carbon atoms or for example 1 to 4 carbon atoms. When a subscript is used herein following a carbon atom, the subscript refers to the number of carbon atoms that the named group may contain. Thus, for example, C₁-₆alkyl means an alkyl of one to six carbon atoms. Examples of alkyl groups are methyl, ethyl, propyl, isopropyl, butyl, isobutyl, sec-butyl, tert-butyl, 2-methylbutyl, pentyl iso-amyl and its isomers, hexyl and its isomers, heptyl and its isomers and octyl and its isomers. Where alkyl groups as defined are divalent, i.e., with two single bonds for attachment to two other groups, they are termed "alkylene" groups. Non-limiting examples of alkylene groups includes methylene, ethylene, methylmethylene, trimethylene, propylene, tetramethylene, ethylethylene, 1,2-dimethylethylene, pentamethylene and hexamethylene.

The term "aryl" as used herein by itself or as part of another group refers but is not limited to 5 to 14 carbon-atom homocyclic (i.e., hydrocarbon) monocyclic, bicyclic or tricyclic aromatic rings or ring systems containing 1 to 4 rings which are fused together or linked covalently, typically containing 5 to 8 atoms; at least one of which is aromatic. Where aryl groups as defined are divalent, i.e., with two single bonds for attachment to two other groups, they are termed "arylene" groups.

The term "C₁-₆alkylcarbonyl" as used herein refers to a group of general formula C₁-₆alkyl-CO-, wherein C₁-₆alkyl is as defined above.

The term "amino" refers to the group -NH₂.

The term "halo" or "halogen" as a group or part of a group is generic for fluoro, chloro, bromo or iodo.

The term "epoxy" or "epoxide" as used herein refers to cyclic ether with only three ring atoms.

The term "C₁-₆alkylarylsulfoxide" as used herein refers to a compound with a C₁-₆alkyl moiety, an aryl moiety and a group of general formula -SO₂.

The term "heteroaryl" as used herein refers to a group of five to about a 14-membered aromatic monocyclic or multicyclic hydrocarbon ring system, including fused and spiro rings, in which one or more of the elements in the ring system is an element other than carbon and is selected from nitrogen, oxygen, silicon, or sulfur and wherein an N atom may be in the form of an N-oxide.

The term "heteroaryloxycarbonylC₁-₆alkyl" as used herein refers to a group of general formula heteroaryl-OC (O)-C₁₋₆alkyl group where R is a C₁₋₆alkyl is a group as previously described heteroaryl group as previously described.

The term "C₁₋₆alkoxy" as used herein refers to a group of general formula -O-C₁₋₆alkyl.

The term "thio" as used herein refers to the -SH group. The term "isocyano" as used herein refers to the group of formula -N=C=O. The term "isothiocyano" as used herein refers to a group of formula -N=C=S.

In an embodiment, the organic molecule (I) is optionally coupled, preferably covalently coupled, to the receptor of formula X-D-Ala-D-Ala using a multifunctional arm-spacer of the general formula Z¹-(CH₂)ᵥ-Z² wherein v is an integer selected from 2 to 12; or Z¹-CH₂-(O-CH₂-CH₂)ₐ-OCH₂-Z² wherein a is an integer selected from 0 to 5; wherein Z¹, Z² are each independently selected from N₃-aryl-; diazirinyl; -CO₂H and N-hydroxysuccinimidyl ester thereof; -CH₂-NH₂ and N-maleimidyl derivative thereof; -CH₂OH and tosylates thereof; -CH₂-SH and dithiane derivatives thereof; -CH₂-N=C=O; -CH₂N=C=S; 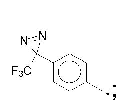 or

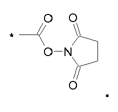

In an embodiment, the organic molecule is covalently coupled with a multifunctional arm-spacer as described above.

In an embodiment, for the binding of the receptor moiety to the organic molecule (I) grafted on the surface of the ATR element or the quartz crystal, N-hydroxysuccinimidyl (NHS) esters are preferred. They are able to covalently fix the X-D-Alanyl-D-Alanine receptors. In a preferred embodiment the N-hydroxysuccinimidyl ester is N₃-C₆H₄-CH₂-CH₂-CH₂-CO-NHS ester.

In an embodiment, the construction of a device comprising a compound grafted on its surface, preferably covalently grafted on its surface, able to immobilize vancomycin, comprises the steps of:
(a1) chemically activating at least part of an ATR element's surface by oxidation, hydroxylation or reduction in acid or alkaline environment, or
(a2) chemically activating at least part of a gold-plated quartz crystal surface by oxidation, hydroxylation or reduction in acid or alkaline environment,
   and
   (b) covalently grafting on said chemically activated surface of step (a1) or (a2) an organic molecule as described above. In an embodiment, said organic molecule is selected from the group comprising HS-(CH₂-CH₂-O)_{w}-CH₃, X¹₃Si-(CH₂)_{q}-NH-CO-(O-(CH₂)ₐ-X¹, CH≡CR⁶, CH₂=CHR⁶, X¹₃Si-(CH₂)_{q}-(CF₂)ₛ-Y¹, X¹₂(R³)Si-(CH₂)_{q}-(CF₂)ₛ-Y¹; or X¹(R³)(R⁴)Si-(CH₂)_{q}-(CF₂)ₛ-Y¹; wherein R³, R⁴, R⁶, X¹ Y¹ , q, and s, have the same meaning as that defined above. In an embodiment, said organic molecule can be optionally further covalently coupled with a multifunctional arm-spacer as described herein above, prior to being reacted to a receptor moiety of formula X-D-Ala-D-Ala able to immobilize vancomycin, thereby giving the compound able to immobilize vancomycin.

In an embodiment, the construction of an FTIR-ATR biosensor comprising molecules covalently grafted on its surface, wherein said molecules are covalently linked to a representative of X-D-Ala-D-Ala receptor moiety can be performed as illustrated in Scheme 1.

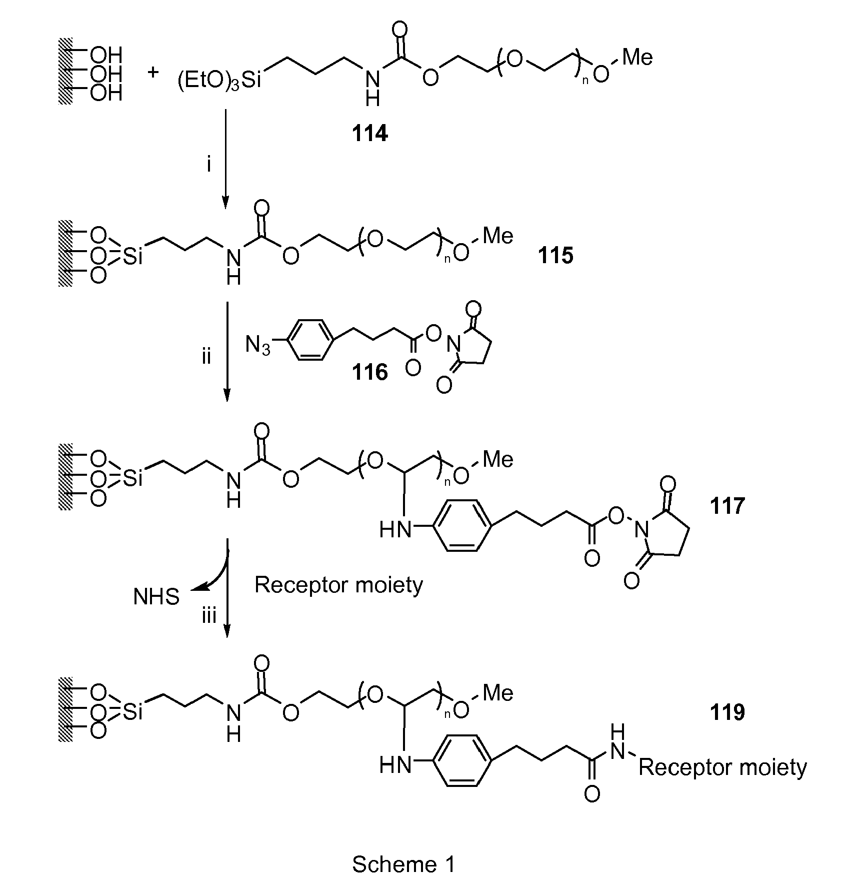

In a preferred embodiment, the construction of the device can be described as follows: at least one surface of ATR element (optical element) is oxidized and then functionalized with organic amphiphilic molecules via a combination of surface wet-chemistry and photochemical processes. For example the optical element is surface-oxidized and then reacted with an organic molecule (I) as described above, for example with compound of formula 114 to obtain grafted device of formula 115. Next, the photografting of a multifunctional arm-spacer of the general formula Z¹-(CH₂)ᵥ-Z² such as N₃-C₆H₄-CH₂-CH₂-CH₂-CO-NHS (compound 116), where NHS means N-hydroxysuccinimidyl ester, gives the NHS-functionalized ATR element of formula 117, on which is fixed a receptor moiety of formula X-D-Ala-D-Ala, thereby providing a receptor-functionalized ATR element of formula 119 ready for immobilizing vancomycin.

The present invention provides a method for the detection and measurement of free vancomycin. The present method is adapted for obtaining protein-unbound vancomycin in biological samples. The present invention therefore also provides a method as described above for the label-free measurement of protein-unbound vancomycin in a biological sample.

To monitor the vancomycin dosage in patients receiving vancomycin therapy, samples of blood, serum, interstitial fluids or other human fluids susceptible of containing vancomycin, are analyzed at regular intervals for vancomycin. In these samples, vancomycin is partly bound to proteins, mainly albumin. Although it is well known that only the free fraction of vancomycin is biologically active, i.e. vancomycin not bound to proteins, only the total fraction of vancomycin is routinely measured by prior art method and taken into consideration for dosage adjustment in clinics.

According to the invention, the sample is treated with an aqueous solution, prior to adding said sample with the biosensor. In an embodiment of the present invention, the treatment of the sample with an aqueous solution in step (b) of the present method is preferably a dialysis step. Dialysis, preferably micro-dialysis allows to isolate the protein-unbound fraction of vancomycin for measurement. This has as advantage over the available commercial methods that the biologically active vancomycin can directly be determined.

In an embodiment, the method is performed in vitro. In another embodiment, the method is performed in vivo.

In a further embodiment, the above described use is for the monitoring and/or measuring of vancomycin in microdialysate. Preferably the microdialysate is derived from a subject receiving a vancomycin therapy by in vivo microdialysis. This has for advantage that the number of sample preparation steps can be reduced. The detection and monitoring of vancomycin levels in the sample can be obtained in a short time interval. Preferably, the invention provides a method as described above for the monitoring and/or measuring of vancomycin in microdialysate from a subject receiving a vancomycin therapy by in vivo microdialysis.

In an embodiment the microdialysis uses a probe which is inserted into tissue in vivo, such that one side of a semi-permeable membrane is in contact with tissue and extra cellular liquid and the other side is flushed or rinsed with a dialysis liquid (perfusate) which takes up substances from the extra cellular liquid through the membrane. A substance can also be distributed locally to the extra-cellular liquid through the perfusion liquid. These substances can then be analyzed in the dialysate on or after exiting the probe. Probes are often made in the form of an inner and an outer tube, where the outer tube exhibits a membrane and the dialysate and the perfusate is entering and exiting the tube at one end and the other end of the tubes are fused or plugged.

Microdialysis has the advantage of allowing the examination of the amounts present or missing of substances in patients. It also allows to monitor changes in the status of substances connected with the used of medicaments.

In a preferred embodiment, the method of the invention is used for the monitoring of a subject being treated with vancomycin; it increases treatment accuracy and reliability. As used herein the term "subject" or "patient" or "individual" refers to any vertebrate species. Preferably, the term subject encompasses warm-blooded vertebrates, more preferably mammals. More particularly contemplated are mammals such as humans, as well as animals such as carnivores other than humans (such as cats and dogs), swine (pigs, hogs, and wild boars), poultry, ruminants (such as cattle, oxen, sheep, giraffes, deer, goats, bison, and camels), and horses.

The inventors established that the treatment aqueous solution should be free of phosphate in order for the protein-unbound vancomycin to be detectable with the above described biosensor. Without being bound to a theory, it is speculated that the presence of phosphate in the detection medium is hampering that vancomycin-receptor bonds are established. This unexpected behavior requires the use of non traditional media for allowing the quantitative analysis of the "antibiotic-receptor" complex by FTIR spectroscopy or by QCM.

By "phosphate" it is meant compounds from the group M¹H₂PO₄, M¹₂HPO₄, M¹₃PO₄, M² (H₂PO₄)₂, M²₃(PO₄)₂ and M²HPO₄, wherein M¹ is Na, Li, or K, and M² is Ca or Mg. Representatives of this group are NaH₂PO₄, KH₂PO₄, Na₂HPO₄, K₂HPO₄, Mg(H₂PO₄)₂.

In a preferred embodiment of the invention, an aqueous solution free of compounds of the list M¹H₂PO₄, M¹₂HPO₄, M¹₃PO₄, M²(H₂PO₄)₂, M²₃(PO₄)₂ and M²HPO4 are used for treating a sample. The treated sample should preferably result in an aqueous solution free of any of the compounds of the list M¹H₂PO₄, M¹₂HPO₄, M¹₃PO₄, M²(H₂PO₄)₂, M²₃(PO₄)₂ and M²HPO₄.

Although phosphate buffered saline is typically used in biological applications for the stabilization of the pH around 7, and it is often the medium of choice in micro-dialysis, the present invention requires that its use be avoided in the above described method.

In an embodiment of the invention, the aqueous solution is also free of salt, preferably free of salts of formula M³X² or M⁴X²₂, wherein X² is selected from F, Cl, Br, I and M³ is selected from Li, Na, K, NH₄ and alkylammoniums; M⁴ is selected from Ca, Mg, Zn, Cu, Fe, Ni, Co. In a preferred embodiment, the solution is free of sodium chloride (NaCl).

The inventors have found that an aqueous medium comprising at least one carbohydrate can at the same time provide an isotonic medium for dialysis and provide a solution that lacks phosphate. By isotonic medium it is meant that the solution has the same osmotic pressure as the fluid it will be immersed in for dialysis, e.g. blood. Use of an isotonic medium for dialysis has for advantage that the perfusate will not force cells, such as blood cells, to take up or loose water. Due to the dialysate having the same osmotic pressure as e.g. blood serum, it can be passed directly into the body of a subject.

The inventors have found that it is advantageous to use an aqueous solution comprising at least one carbohydrate. The term "carbohydrate" as used herein includes monosaccharides, oligosaccharides and polysaccharides as well as substances derived from monosaccharides by reduction of the carbonyl group such as alditols, by oxidation of one or more terminal groups to carboxylic acids, or by replacement of one or more hydroxy group(s) by a hydrogen atom, an amino group, a thiol group or similar heteroatomic groups. It also includes derivatives of these compounds.

The carbohydrate may be composed of 1 to 6 carbohydrate units. According to an embodiment said carbohydrate constituent is composed of 1 to 4 carbohydrates units. Suitable carbohydrates include but are not limited to mono- oligo and poly-saccharides. In an embodiment, said at least one carbohydrate is selected from the group comprising glucose, fructose, lactose, galactose, mannose, maltose, nystose, kestose, trehalose, raffinose, gentianose, ribose, arabinose, erythose, threose, lyxose, xylose, sorbitol, allose, altrose, talose, gulose, idose, ribulose, xylulose, psicose, sorbose, tagatose, saccharose, cellobiose, or mixture thereof. Preferably said at least one carbohydrate is glucose.

In an embodiment, the invention provides a method as described above wherein said aqueous solution is an aqueous solution comprising from 1 to 10 w% of at least one carbohydrate, for example from 3 to 7 wt% of at least one carbohydrate, preferably 5 wt% of at least one carbohydrate. For example, said aqueous solution can comprise from 1 to 10 w% of glucose, for example from 3 to 7 wt% of glucose. In a preferred embodiment, said aqueous solution is a solution comprising 5 wt% of glucose in water.

The invention also provides a method as described above for the detection and quantitative analysis of vancomycin utilizing the receptor moiety of formula X-D-Alanyl-D-Alanine immobilized on a FTIR-ATR optical element, or on a gold-plated quartz crystal, in an isotonic solution of weak ionic strength, preferably an aqueous solution comprising 1 to 10 wt% of at least one carbohydrate in water, preferably 5 wt% of glucose in water.

In a preferred embodiment, the method as described above is used for the label-free measurement of protein-unbound vancomycin in a biological sample, wherein said biological sample lacks phosphates, preferably wherein said biological sample lacks any of the compounds selected from the group M¹H₂PO₄, M¹₂HPO₄, M¹₃PO₄, M²(H₂PO₄)₂, M²₃(PO₄)₂ and M²HPO₄, wherein M¹ is Na, Li, or K, and M² is Ca or Mg.

In a further embodiment, the invention provides a method as described above for monitoring and/or measuring vancomycin in the treatment of an infection caused by Gram positive micro-organisms in a subject in need thereof.

In a further embodiment, the invention provides a method as described above, wherein the infection is due to methicillin-resistant *Staphylococcus aureus* (MRSA).

According to this embodiment, the Gram positive micro-organism is preferably methicillin-resistant *Staphylococcus aureus* (MRSA). MRSA is a bacterium responsible for several difficult-to-treat infections in humans. By the term MRSA any strain of *Staphylococcus aureus* bacteria is meant that has developed resistance to beta-lactam antibiotics, which include the penicillins, such as methicillin, dicloxacillin, nafcillin, and oxacillin; and the cephalosporins. Synonyms for MRSA are multi-drug resistant *Staphylococcus aureus* or oxacillin-resistant *Staphylococcus aureus* (ORSA). MRSA is especially troublesome in hospitals, where patients with open wounds, invasive devices and weakened immune systems are at greater risk of infection than the general public. Techniques for the identification and characterization of the type of bacteria that is causing an infection are well known to a person skilled in the art.

In a further embodiment, the invention provides a method as described above for the detection and quantitative analysis of vancomycin utilizing the molecular moiety X-D-Alanyl-D-Alanine immobilized on a FTIR-ATR optical element, or on a gold-plated quartz crystal, and a particular medium for the direct, label-free, spectroscopic measurement of this interaction, in an isotonic solution of weak ionic strength as described above.

The present invention can be further illustrated by the following examples, although it will be understood that these examples are included merely for purposes of illustration and are not intended to limit the scope of the invention unless otherwise specifically indicated.

### Examples

### Example 1: Construction of a biosensor for vancomycin

Example 1 illustrates the preparation of a vancomycin biosensor according to an embodiment of the present invention.

A germanium ATR element, preferably a small bar of triangular section (28) (h = 0.3 mm; I = 45 mm), is surface-oxidized as follows. The germanium device was washed with 38% HNO₃ (1 min) and rinsed with MilliQ water. Oxidation was performed with 99+% oxalic acid / 35% H₂O₂ (10/90, v/v) during 5 min at 20°C, and washing with MilliQ water. This was repeated three times. The device was dried under N₂ flux (17 h).

The device is then reacted with (EtO)₃Si-CH₂-CH₂-CH₂-NH-CO-(O-CH₂-CH₂)₇-OCH₃ as follows. The device was immersed into 2% silane solution in CCl₄, under N₂ atmosphere, during 3h at reflux. The crystals were washed with methanol (2h) and with tetrahydrofuran (2h) in a Soxhlet apparatus.

Next, the surface was photografted with N₃-C₆H₄-CH₂-CH₂-CH₂-CO-NHS, named molecular clip. NHS stands for N-hydroxysuccinimidyl ester. The above described procedure resulted in an NHS-functionalized ATR element. For photografting the azide, a solution of molecular clip in benzene (5 mg/1.5 mL) was sprayed on the device and the solvent was evaporated under air flux in the dark to obtain a deposition of 0.1 to 0.2 mg / cm². The crystal was submitted to UV irradiation during 2 h at room temperature (3 lamps of 8 W and λₘₐₓ 254 nm, placed at a distance of 10 cm). The device was rinsed with THF (10 min) and CHCl₃ (5 min) under shaking at 20°C (Edmund Bühler stirrer, model KL-2, 150 rpm), and provided the activated surface.

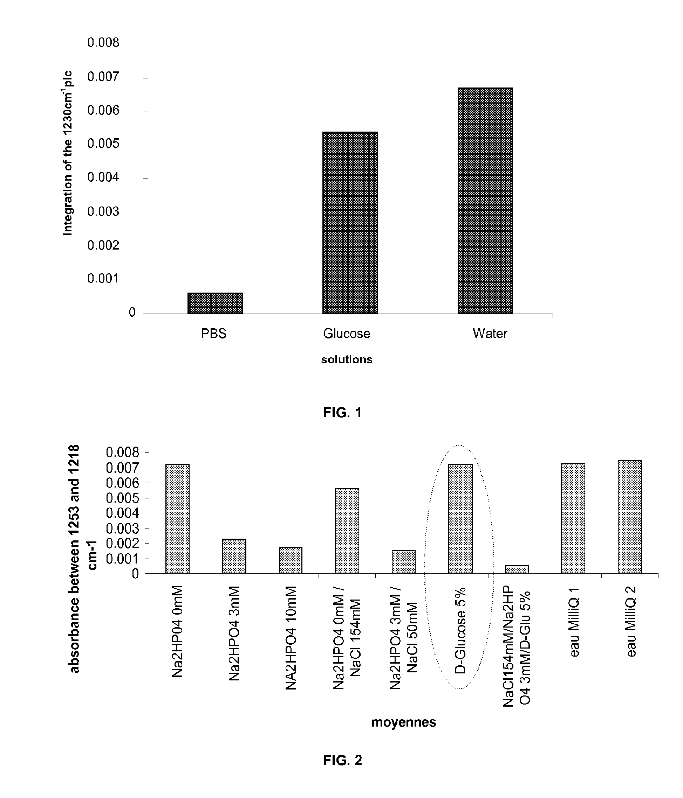

On the thus functionalized ATR element a peptide was bound. Peptide binding onto the device was obtained in one channel of measurement at a time by incubation for 3 hours of N-α-acetyl-Lys-D-Ala-D-Ala peptide, or another peptide as provided in Figure 2, at a concentration of 2 mg/mL in phosphate buffered saline (PBS) at room temperature. The measurement channel was then rinsed with milliQ water for 30 min.

The binding of the peptide was monitored by FTIR-ATR spectroscopy by using the BIA-ATR device. Attenuated Total Reflection Fourier Transformed Infrared (ATR-FTIR) Spectra were obtained on a Brüker IFS55 FTIR spectrophotometer (Ettlingen, Germany) equipped with a MCT detector (broad band 12000-420 cm⁻¹, liquid N₂ cooled, 24h hold time) at a resolution of 8 cm⁻¹ with an aperture of 3.5 mm and acquired in the double-sided, forward-backward mode. Two levels of zero filling of the interferogram prior to Fourier transform allowed encoding the data every 1 cm⁻¹. The spectrometer was continuously purged with dry air (Whatman 75-62, Haverhill, MA, USA). Triangular-shaped germanium crystals (4.8 x 4.8 x 45 mm³) were purchased from Biosentech (Belgium) and accommodated on the beam condenser from a Golden Gate Micro-ATR from Specac. A top plate with a groove fitting the crystal was used in replacement of the diamond-bearing plate (WOW Company, Belgium). With this geometry, a single reflection was obtained and more than 10 lanes can be used on a crystal.

A quantitative analysis of binding rates of the receptors thus constructed was obtained by recording the surface of the amide bonds in function of time. The intensity of these signals allowed the normalization of the binding results by referring the amount of bound ligand to the amount of immobilized receptor.

### Example 2: Detection and quantitative measurement of vancomycin in water

Using a biosensor constructed in Example 1, a solution of vancomycin in pure water (obtained from the laboratory MilliQ system) at a concentration of at 50 µg/mL is passed over one channel of the crystal at a flow-rate of 20 µl/min. FTIR-ATR spectra were recorded continuously and the vancomycin binding is monitored by following the appearance of its characteristic signal at 1230 cm⁻¹. This monitoring of this signal is advantageous as it is isolated in the spectra, i.e. not superimposed to other signals due to ligand and/or receptor. The intensity of the peak at 1230 cm⁻¹ thus allowed the quantitative measurement of vancomycin binding.

Similar experiments have been performed using the other X-D-Ala-D-Ala biosensors and various concentrations of vancomycin solutions (from 0 to 200 µg vancomycin /mL water). Vancomycin binding curves *versus* the different receptors were quite similar. This allowed the quantitative analysis of vancomycin solutions in the range of 0 to 100 µg/mL.

### Example 3: Detection and quantitative measurement of vancomycin in various aqueous media

The N-α(Ac)-L-Lys-D-Ala-D-Ala biosensor as described in Example 1 was used. A solution of vancomycin in phosphate buffered saline (50 µg/mL) was used in the binding assay. The quantitative analysis was performed by measuring the surface on the IR spectra comprised between 1253 and 1218 cm⁻¹. The spectra were recorded every 30 sec during 5 min. Surprisingly, the amount of vancomycin detected was very weak compared to the result recorded in pure water. The results are depicted in Figure 1.

The unexpected effect on the binding affinity of vancomycin *versus* X-D-Ala-D-Ala receptor immobilized on the ATR optical element, has been further observed by using the following detection media: 3 mM aqueous solution of Na₂HPO₄, 10 mM aqueous solution of Na₂HPO₄, 154 mM aqueous solution of NaCl, and a PBS solution made from 3 mM Na₂HPO₄ and 50 mM NaCl. The results are displayed in Figure 2. From the results depicted in Figure 2, it was concluded that phosphate totally prevents the vancomycin binding while sodium chloride at physiological concentration partially inhibited the binding.

### Example 4: Detection and quantitative measurement of vancomycin in 5% glucose solution

The Nα(Ac)-L-Lys-D-Ala-D-Ala biosensor described in Example 1 was used. The assays were performed three times successively using the same channel, and this has been repeated at three different days (n = 9) using another channel for each day. Solutions of vancomycin in 5% glucose at various concentrations (from 0 to 25 µg/mL) were passed over the crystal at a flow rate of 20 µL/min. The vancomycin binding was analyzed by the integration of the peak at 1230 cm⁻¹ (10 spectra recorded every 30 sec), and normalized by referring to the 25 µg/mL concentration.

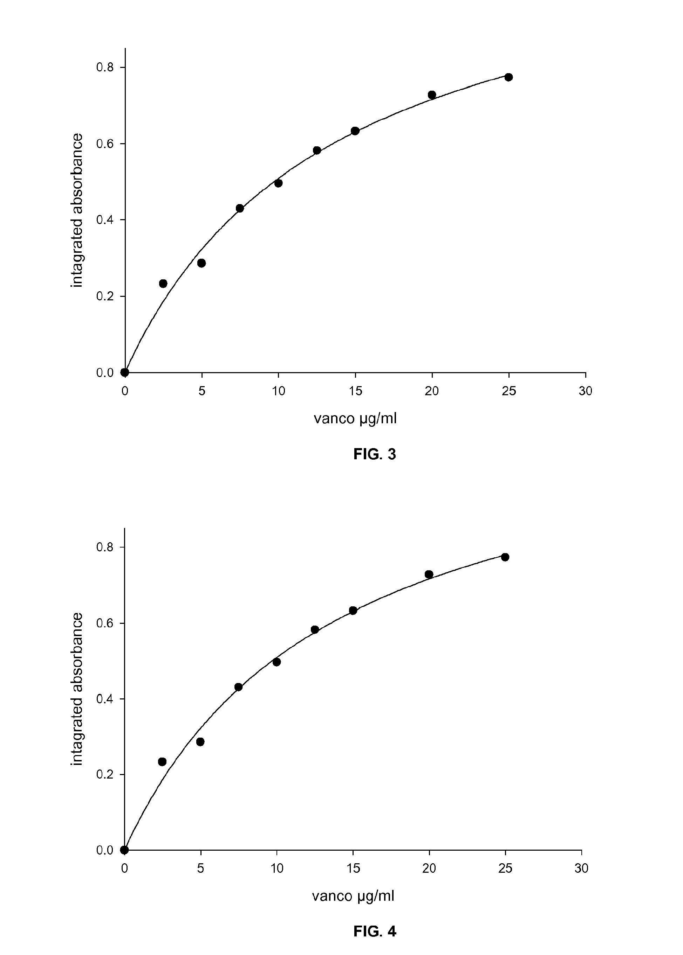

As reported in figure 2, vancomycin binding onto the receptor is maximal when present in pure water. The presence of phosphate buffer molecules in the solution dramatically decreased the binding level. Phosphate concentrations as low as 3 mM did not- allow sufficient vancomycin binding. Furthermore, presence of NaCl also reduced significantly vancomycin binding. For instance 150 mM NaCl alone (not shown) dramatically reduced vancomycin binding onto the receptor. In an unexpected manner, 5% glucose in water as detection medium resulted in detection levels similar to those observed for pure water. Yet, 5% glucose combined with NaCl (154 mM) and Na₂HPO₄ (3 mM) were poorly efficient, as can be seen for the results displayed in Figure 3.

As shown in Figure 3, even a concentration as low as 2.5 µg/mL was easily detected in 5% glucose. The experimental error in the range of the clinically relevant concentration of 10-25 µg/mL was lower than ± 5 µg/mL.

### Example 5: Detection and quantitative measurement of vancomycin in human serum samples.

Human serum was dialyzed against 5% glucose. For the serum dialysis a commercially available microdialysis system was used (CMA 20 Elite, CMA Microdialysis, Sweden). The system comprised a membrane of polyarylethersulfone with a length of 10 mm and a cut-off of 20,000 Dalton.

The perfusate obtained was spiked with 0, 2.5, 5, 7.5, 10, 12.5, 15, 20 and 25 µg/mL vancomycin respectively.

The spiked dialyzed serum samples were flown over a measurement channel on which the Nα(Ac)-L-Lys-D-Ala-D-Ala had been attached as described before in Example 1. The absorption band between 1259 and 1213 cm⁻¹ was quantified for each vancomycin concentration. The area of the band was reported as a function of vancomycin concentration. The results are displayed in Figure 4. From the data reported in Figure 4 it follows that vancomycin binding can be monitored with precision in a concentration range which is relevant for medical application.

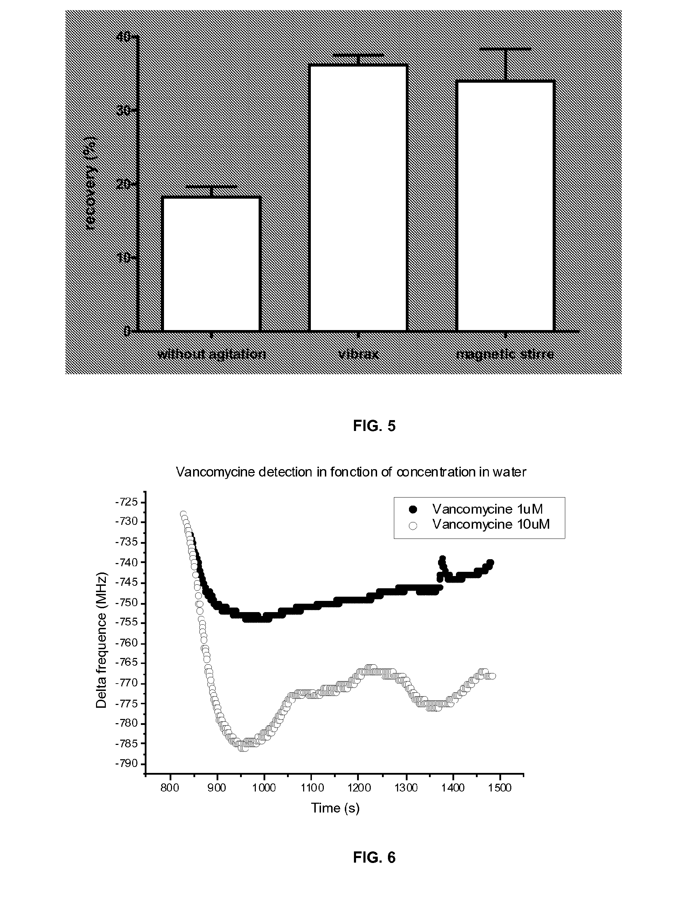

Special attention must be paid to the quantitative recovery of the free vancomycin drug. This was ensured by first allowing a sufficient equilibration time of about 30 min at about 37°C, adjusting the flow rate to 2 µL/min, and by careful agitation of the fluid in which the probe of the microdialysis system was inserted. The flow rate of a blood vessel was modeled using a mechanical vibrator. Alternatively it can be achieved by ensuring a sufficient flow rate of the fluid such as would occur in vivo in a blood level. A stable and reproducible recovery of about 40 % was achieved, as displayed in Figure 5. A stable value of about 36 % was obtained with the mechanical vibrator "Vibrax" (Ika-vibrax-VXR electronic Type VX2, speed of 200 rpm).

### Example 6: Quartz crystal microbalance (QCM) experiment

Quartz crystal microbalance experiments were performed using SRS Stanford Research Systems (QCM 200 5MHz). The surfaces of gold-plated quartz crystal on chips were prepared as follows: gold-plated quartz crystals were initially immersed in a piranha solution (70/30) for 3 minutes to regenerate the surfaces. The resulting crystals were repeatedly and intensively rinsed with water. The crystals were subsequently grafted with mPEG thiols (O-(2-Mercaptoethyl)-O'-methyl-hexa(ethylene glycol) (polypure) 10⁻³ M in ethanol. The grafted crystals were subsequently rinsed with ethanol in an ultrasonic bath, and dried with N₂. The grafted crystals were subsequently grafted with NHS ester succinimidyl, 2 mM in acetonitrile sprayed on the surfaces and the surfaces were placed in front of a UV lamp during 2 hours. The functionalized surfaces of the crystals were subsequently rinsed with acetonitrile in an ultrasonic bath; dried with N₂, and then subsequently grafted with N-α-acetyl-Lys-D-ala-D-ala in pure water and in water with 5% of glucose (vol.).

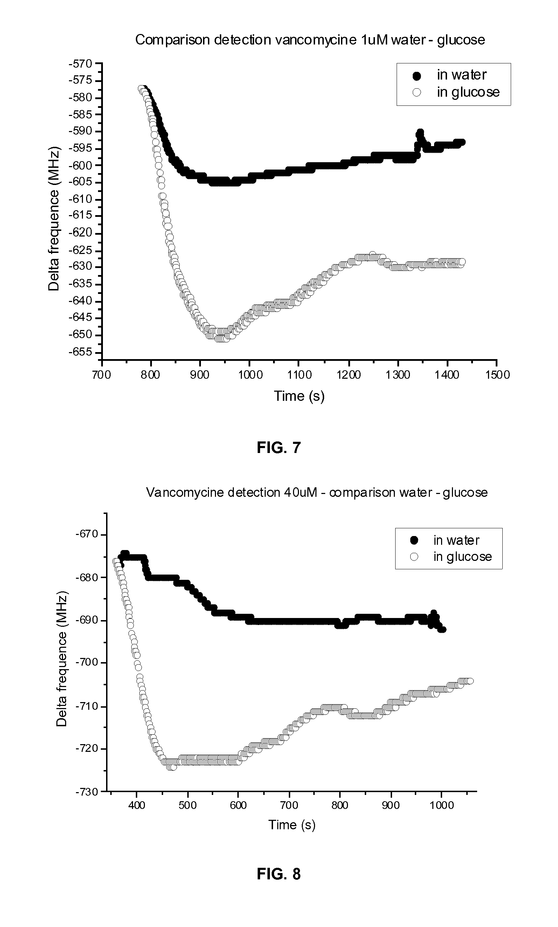

All QCM experiments used a flow rate of 40 µL/min. The quality of all the grafting has been tested at each step, by contact angle measurements and IR spectra. All of them were satisfactory. Figures 6, 7 and 8 represent QCM data plot showing delta frequency (MHz) change over time of vancomycin detection. Solutions of water or water-glucose at different concentrations were exposed to QCM measurements using the flow injection system at the given flow rate.

The results of the experiment are shown in Figures 6, 7 and 8, where a shift in the QCM response at equilibrium on the sensor surface was observed. When comparing the shift with water or with water/glucose solution, greater delta frequency was always observed in the presence of glucose.

## Claims

1. Method for detecting and measuring free vancomycin in a sample, comprising the steps of:
(a) providing a device comprising at least one surface which is chemically activated and grafted with a compound able to immobilize vancomycin,
(b) providing a sample, treating said sample with an aqueous solution and adding said treated sample to said device, and
(c) measuring the free vancomycin concentration in said treated sample,
wherein said aqueous solution comprises at least one carbohydrate and is free of any of the compounds selected from the group M¹H₂PO₄, M¹₂HPO₄, M¹₃PO₄, M²(H₂PO₄)₂, M²₃(PO₄)₂ and M²HPO₄, wherein M¹ is Na, Li, or K, and M² is Ca or Mg.

2. Method according to claim 1, wherein said device comprises an attenuated total internal reflection element, transparent in the infra-red.

3. Method according to claim 1, wherein said device is a quartz crystal microbalance.

4. Method according to any of claims 1 to 3, wherein said treatment in step (b) is a dialysis, preferably a micro-dialysis.

5. Method according to any of claims 1 to 4, wherein said aqueous solution is an aqueous solution comprising from 1 to 10% wt% at least one carbohydrate, preferably from 3 to 7 wt% at least one carbohydrate, preferably 5 wt% of at least one carbohydrate.

6. Method according to claim 1 to 5, wherein said at least one carbohydrate is selected from the group comprising glucose, fructose, lactose, galactose, mannose, maltose, nystose, kestose, trehalose, raffinose, gentianose, ribose, arabinose, erythose, threose, lyxose, xylose, sorbitol, allose, altrose, talose, gulose, idose, ribulose, xylulose, psicose, sorbose, tagatose, saccharose, cellobiose, or mixture thereof, preferably said at least one carbohydrate is glucose.

7. Method according to any of claims 1 to 6, wherein said solution is further free of salts of formula M³X² or M⁴X²₂, wherein X² is selected from F, Cl, Br, I and M³ is selected from Li, Na, K, NH₄ and alkylammoniums; M⁴ is selected from Ca, Mg, Zn, Cu, Fe, Ni, Co.

8. Method according to any of claims 1 to 7, wherein the sample is serum, blood, or interstitial fluid.

9. Method according to any of claims 1 to 8, wherein said compound able to immobilize vancomycin comprises a receptor of the general formula X-D-Alanyl-D-Alanine, wherein
X is NH₂[(CH₂)ₘ-O]_{y}-(CHR¹)_{z}-CO-L¹-,
m is an integer selected from 1 to 6,
y is an integer selected from 0 and 10, preferably 0, 3 or 6,
z is an integer selected from 1 to 10, preferably 1,
R¹ is H or NHR², R² is H or C₁-C₆ alkylcarbonyl,
L¹ is a single bond or -NH-(CH₂)ₙ-CO-, wherein n is an integer selected from 1 to 8, preferably 5, preferably X is L-Lysyl, or a protected form thereof, preferably X is N-α-acetyl-L-lysyl.

10. Method according to claim 9, wherein the receptor of formula X-D-Alanyl-D-Alanine, is grafted on the surface of the device, via an organic molecule selected from the group comprising HS-(CH₂-Ch₂-O)_{w}-CH₃, X¹₃Si-(CH₂)_{q}-NH-CO-(O-(CH₂)ₛ-X¹, CH≡CR⁶,CH₂=CHR⁶, X¹₃Si-(CH₂)_{q}-(CF₂)ₛ-Y¹, X¹₂(R³)Si-(CH₂)_{q}-(CF₂)ₛ-Y¹; or X¹(R³)(R⁴)Si-(CH₂)_{q}-(CF₂)ₛ-Y¹ , wherein
R⁶ is selected from C_{w}H_{2w+1}, C_{w}F_{2w+1}, -(CH₂)ᵤ-(O-CH₂-CH₂)ₚ-OR⁵, wherein R⁵ is selected from C₁-₄alkyl, C₁-₆alkylarylsulfoxide, heteroaryloxycarbonylC₁₋₆alkyl, 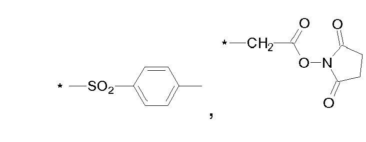 , or mixture thereof,
w is an integer from 3 to 50,
u is an integer from 0 to 20,
p is an integer from 3 to 20;
X¹ is halogen or C₁-₆alkoxy;
q is an integer selected from 1 to 20;
s is an integer selected from 0 to 20;
R³ and R⁴ are each independently C₁-₆alkyl; and
Y¹ is Me, CF₃, CHF₂, CH₂F, CH=CH₂, CN, CH=O, epoxide, halogen, SH, NH₂, OH, N=C=O, N=C=S, CO₂H or derived esters thereof

11. Method according to claim 10, wherein the organic molecule is optionally coupled to the receptor of formula X-D-Ala-D-Ala using a multifunctional arm-spacer of the general formula Z¹-(CH₂)ᵥ-Z² wherein v is an integer selected from 2 to 12; or Z¹-CH₂-(O-CH₂-CH₂)a-OCH₂-Z² wherein a is an integer selected from 0 to 5; wherein Z¹, Z² are each independently selected from N₃-aryl-; diazirinyl; -CO₂H and N-hydroxysuccinimidyl ester thereof; -CH₂-NH₂ and N-maleimidyl derivative thereof; -CH₂OH and tosylates thereof; -CH₂-SH and dithiane derivatives thereof; -CH₂-N=C=O; -CH₂N=C=S; 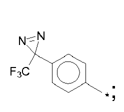 or 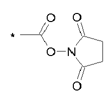 , preferably said multifunctional arm-spacer is an N-hydroxysuccinimidyl ester spacer, preferably N₃-C₆H₄-CH₂-CH₂-CH₂-CO-N-hydroxysuccinimidyl ester.

12. Method according to any of claims 1, 2, 4 to 11, wherein the free vancomycin concentration is measured by Fourier Transform Infra Red (FTIR)-ATR spectroscopy.

13. Method according to any of claims 1 to 12, for the monitoring of vancomycin in microdialysate from a subject receiving a vancomycin therapy by in vivo microdialysis.

14. Method according to any of claims 1 to 13, for monitoring and/or measuring vancomycin in the treatment of an infection caused by Gram positive micro-organisms in a subject in need thereof.

15. Method according to claim 14, wherein the infection is due to methicillin-resistant *Staphylococcus aureus* (MRSA).
